(19) Europäisches Patentamt
European Patent Office
Office européen des brevets

(11) Veröffentlichungsnummer: **0 022 744**
**B1**

(12) # EUROPÄISCHE PATENTSCHRIFT

(45) Veröffentlichungstag der Patentschrift: **23.03.83**

(51) Int. Cl.³: **C 07 C 103/78**, C 07 C 103/46, A 61 K 49/04

(21) Anmeldenummer: **80730043.9**

(22) Anmeldetag: **27.06.80**

(54) **Trijodierte Basen, deren Herstellung und diese enthaltendes Röntgenkontrastmittel.**

(30) Priorität: **12.07.79 DE 2928417**

(43) Veröffentlichungstag der Anmeldung: **21.01.81 Patentblatt 81/3**

(45) Bekanntmachung des Hinweises auf die Patenterteilung: **23.03.83 Patentblatt 83/12**

(84) Benannte Vertragsstaaten: **AT BE CH DE FR GB IT LI LU NL SE**

(56) Entgegenhaltungen: **DE-A-2 547 789**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Speck, Ulrich, Dr., Benediktiner Strasse 50, D-1000 Berlin 28 (DE)**
Erfinder: **Klieger, Erich, Dr., Augsburger Strasse 25, D-1000 Berlin 30 (DE)**
Erfinder: **Mützel, Wolfgang, Dr., Weddigenweg 74, D-1000 Berlin 45 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 022 744 B1

BUNDESDRUCKEREI BERLIN

# Trijodierte Basen, deren Herstellung und diese enthaltendes Röntgenkontrastmittel

Die vorliegende Erfindung umfaßt die in den Ansprüchen gekennzeichneten Gegenstände.

Zur vollständigen röntgenologischen Darstellung von z. B. wesentlichen Teilen des Gefäßsystems, wie beispielsweise der Aorta, oder der vollständigen Darstellung der ableitenden Harnwege müssen Kontrastmittellösungen in hohen Konzentrationen appliziert werden, um einen guten Kontrast in dem zu untersuchenden System zu erhalten. Die hohen Kontrastmittelkonzentrationen erfordern von den Kontrastmitteln eine sehr gute Wasserlöslichkeit bei gleichzeitig niedriger Toxizität und guter Verträglichkeit.

Es ist bekannt, daß die allgemeine Verträglichkeit der Röntgenkontrastmittel von deren hydrophilen Charakter abhängig ist (P. K. Knoefel, Radiocontrast agents, Vol. Pergamon Press 1971, page 133 ff.) und daß die elektrische Ladung des Kations und Anions zu einem wesentlichen Teil zur Verminderung der lipophil-toxischen Wirkungen insbesondere der trijodierten Aromaten beiträgt.

Bei den zur Zeit gebräuchlichen Röntgenkontrastmittelsalzlösungen ist das Anion der Jod-enthaltende, kontrastgebende Teil, während das Kation keine für die Röntgendarstellung erforderliche Aufgabe erfüllt. Von den verwendeten Kationen gehen im Gegenteil eine Reihe von unerwünschten Wirkungen aus:

So tragen alle Ionen zum osmotischen Druck der Röntgenkontrastmittel-Salzlösungen im Verhältnis ihrer molaren Konzentration bei, d. h. zum Beispiel, daß in den Salzen der substituierten Trijodbenzoesäuren die Hälfte des osmotischen Druckes vom jodfreien und damit kontrastwirkungslosen Kation herrührt. Der hohe osmotische Druck konzentrierter Röntgenkontrastmittellösungen ist für eine Reihe unerwünschter pharmakologischer Effekte verantwortlich, wie zum Beispiel für Vasodilation, Schmerz sowie für diuretische Wirkung und damit Verdünnung des ausgeschiedenen Kontrastmittels in der Urographie. Ferner erhöhen die üblichen Kationen aus jodfreien Basen mit hohem Molekulargewicht, wie beispielsweise Meglumin, Glucamin, Lysin, Arginin u. a., die Viskosität der Kontrastmittellösungen beträchtlich.

Kationen mit geringerem Molekulargewicht erhöhen die Viskosität der Kontrastmittellösungen zwar nur wenig, wirken dafür aber toxisch. So wird eine Schädigung der Blut-Hirn-Schranke und Auslösung von Gefäßschmerz durch Na-Ionen und eine höhere Toxizität von Äthanolamin (EVILL, C. A. und G. T. BENNESS: Metrizamide as a non-ionic urographic agent, a comparison with sodium iothalamate and its dimer, Invest Radiol 9: 434—437 (1974), HILAL, S. K.: Trends in preparation of new Angiographic contrast media with special emphasis on polymeric derivatives, Invest Radiol 5: 458—468 (1970)) beschrieben.

Die zur Kationenbildung geeigneten erfindungsgemäßen Verbindungen zeigen einerseits die Vorteile der vergleichbaren bisher zur Kationenbildung verwendeten Verbindungen und vermeiden andererseits deren Nachteile.

Sie zeichnen sich durch folgende Eigenschaften aus:

Die elektrische Ladung als Träger hydrophiler Eigenschaften und als Ursache für die außerordentlich gute Löslichkeit der Trijodaromaten wird beibehalten.

Der osmotische Druck der Kontrastmittellösungen wird bedeutend vermindert, weil der vom kontrastlosen Kation stammende Anteil am osmotischen Druck entfällt.

Die Viskosität wird bedeutend vermindert, da bei gleicher Jodkonzentration die Salzkonzentration erheblich gesenkt wird. Das erlaubt die Herstellung hoch konzentrierter und dennoch sehr wenig visköser Lösungen. Eine geringere Viskosität der Lösungen erleichtert deren Applikation und wirkt sich in der selektiven Angiographie insofern günstig aus, als die Mikrocirculation weniger beeinträchtigt wird als durch visköse Lösungen.

Erfindungsgemäß kommen als Basen 2,4,6-Trijodisophthalsäurederivate in Betracht, die die erforderliche niedrigte Toxizität aufweisen und geeignet sind mit Kontrastmittelsäuren leicht wasserlösliche Salze zu bilden.

Besonders geeignet sind die neuen Verbindungen der allgemeinen Formel I

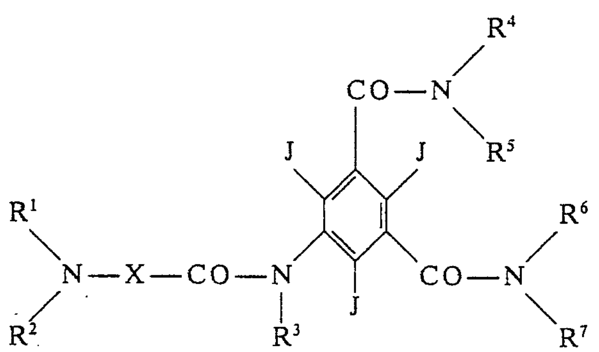

(I)

worin bedeuten

X einen gerad- oder verzweigtkettiger, gegebenenfalls durch eine Hydroxy-, Methoxy- oder Aminogruppe substituierter Alkylenrest mit 1 bis 4 Kohlenstoffatomen,
$R_1$ und $R_3$ Wasserstoff oder ein Alkylrest mit 1 bis 4 Kohlenstoffatomen,
$R_2$ Wasserstoff oder ein hydroxylierter Alkylrest mit 1 bis 4 Kohlenstoffatomen,
$R_4$ und $R_6$ Wasserstoff oder ein gegebenenfalls hydroxylierter Alkylrest mit 1 bis 4 Kohlenstoffatomen,
$R_5$ und $R_7$ ein Mono- oder Polyhydroxyalkylrest mit 1 bis 4 Kohlenstoffatomen,
und deren Salze mit Kontrastmittelsäuren.

Die Alkylgruppen können gerad- oder verzweigtkettig sein. Bevorzugt ist der Methylrest. In Frage kommen aber auch Alkylgruppen, wie beispielsweise die Äthyl-, n-Propyl- oder iso-Propylgruppe.

Sind diese hydroxysubstituiert, so können auch diese geradkettig oder verzweigtkettig sein. Sie enthalten 2–4, vorzugsweise 2–3 C-Atome und 1–3, vorzugsweise 2 Hydroxylgruppen im Alkylrest. Als geeignete Hydroxyalkylreste seien beispielsweise genannt: 2-Hydroxy-propyl, 3-Hydroxy-propyl, 1-(Hydroxymethyl)-äthyl, 2,3-Dihydroxybutyl, 2,4-Dihydroxybutyl, 3,4-Dihydroxybutyl, 2,3-(Dihydroxymethyl)-propyl, (Trishydroxymethyl)-methyl, 2,3,4-Trihydroxybutyl, 1,3,4-Trihydroxybutyl und vorzugsweise 2-Hydroxyäthyl, 2-Hydroxy-1-(hydroxymethyl)äthyl, 2,3-Dihydroxypropyl.

Der Aminoacylrest $R^1, R^2 \cdot N-X-CO-$ ($R^1$, $R^2$ und X haben die oben angegebene Bedeutung) leitet sich von an sich bekannten synthetischen und natürlichen Aminosäuren ab. Bevorzugte Reste X sind gerad- oder verzweigtkettige Alkylenreste mit 1–4, insbesondere 1–2 C-Atomen, wie zum Beispiel Methylen, Äthylen, Propylen, Butylen, Methyl-methylen, Methyl-äthylen, Methyl-propylen, Methyl-butylen und Äthyl-propylen, Hydroxyäthylen, Hydroxymethyl-methylen, Methoxymethyl-methylen.

Die erfindungsgemäßen Verbindungen gemäß Formel I werden insbesondere auch verwendet, um Röntgenkontrastmittelsäuren in ihre Salze zu überführen.

Zur Salzbildung sind grundsätzlich alle bekannten intravenös anwendbaren Kontrastmittelsäuren geeignet. Bevorzugt seien die zur Zeit für die Uro- und Angiographie und die Darstellung der Körperhöhlen gebräuchlichen jodhaltigen Kontrastmittelsäuren genannt wie zum Beispiel einkernige trijodierte Benzoesäuren wie Amidotrizoesäure, Jodamid, Iothalamsäure, Ioxithalamsäure, Ioglicinsäure u. a.

Zweikernige hexajodierte Monocarbonsäuren wie zum Beispiel Ioxaglicinsäure u. a. und zweikernige hexajodierte Dicarbonsäure wie zum Beispiel Iocarminsäure u. a.

Die erfindungsgemäßen Salze, die aus einem trijodierten Kation und einem bekannten jodierten Kontrastmittelsäureanion bestehen, zeichnen sich durch die von Salzlösungen bekannte Stabilität und eine hervorragende Verträglichkeit aus, wie sich aus der nachfolgenden Tabelle am Beispiel des erfindungsgemäßen 5-Aminoacetamido-2,4,6-trijod-isophthalsäure-bis-(2-hydroxyäthyl)amid-Iothalamat (A-Iothalamat) und des erfindungsgemäßen 5-Amidoacetamido-2,4,6-isophthalsäure-bis-(N-methyl-2,3-dihydroxypropyl)-diamid-Amidotrizoat (B-Amidotrizoat).

Tabelle I

| | A-Iothalamat | Na-Iothalamat | Megl. Iothalamat | B-Amidotrizoat | Megl. Amidotrizoat |
|---|---|---|---|---|---|
| Jodgehalt des Salzes | 58% | 60% | 47% | 54% | 47% |
| Viskosität der Lösung mit 300 mg Jod/ml, 37°C in cP | 2,4 | 2,7 | 5,2 | 3,5 | 5,0 |
| Molarität der Lösung mit 300 mg Jod in Mol/Liter | 0,394 | 0,787 | 0,787 | 0,394 | 0,787 |
| $DL_{50}$ Maus (20 g) g Jod/kg | $\geqslant$10,5 | 8,0 | 4,5 (4,1–4,9) | – | – |
| $DL_{50}$ Ratte (100 g) g Jod/kg | $\geqslant$10 | 7,0 | 7,4 (6,8–7,9) | $\geqslant$10 | 7,5 |

Die $DL_{50}$ an Maus und Ratte wurde nach intravenöser Injektion der 300 mg Jod/ml enthaltenden Lösungen mit einer Injektionsgeschwindigkeit von 2 ml/min bestimmt.

So zeigt die obige Tabelle am Beispiel des erfindungsgemäßen Iothalamatsalzes, daß sich die neuen Verbindungen durch einen besonders hohen Jodgehalt, wie er sonst nur für die weniger gut verträglichen Natriumsalze erreicht wird, auszeichnen. Ferner ist zu sehen, daß die Viskosität einer Lösung der erfindungsgemäßen Salze besonders niedrig ist und daß die Molarität und damit der osmotische Druck der Salzlösung wesentlich geringer ist als die Molarität und der osmotische Druck von Na- und Megluminsalzlösungen. Auch ist die Verträglichkeit der erfindungsgemäßen Verbindungen bei Maus und Ratte eindeutig besser als diejenige der bekannten Natrium- oder Megluminsalze.

Die ausgezeichnete Verträglichkeit der neuen Salze in Verbindung mit ihrer niedrigen Viskosität ermöglicht es, hochkonzentrierte und trotzdem leicht injizierbare Röntgenkontrastmittellösungen herzustellen. Gemäß der Erfindung können schattengebende Substanzen in Form ihrer Salzlösungen in Konzentrationen von 20 bis 500 mg J/ml Lösung Anwendung finden.

Die Erfindung betrifft ferner ein Verfahren zur Herstellung der Verbindungen der allgemeinen Formel I, dadurch gekennzeichnet, daß man in an sich bekannter Weise

a) eine Verbindung der allgemeinen Formel II

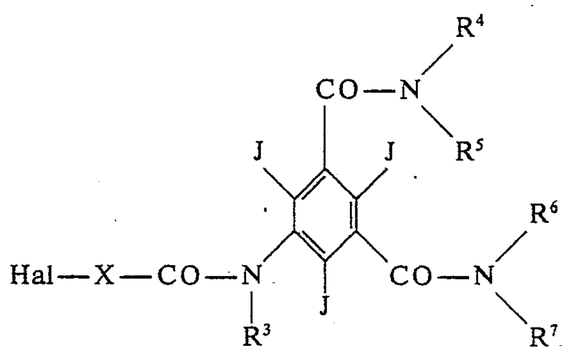

(II)

worin $R^3$ bis $R^7$ und X die oben angegebene Bedeutung haben und Hal ein Chlor- oder Bromatom darstellt, mit einem Amin der allgemeinen Formel III

$$R^1R^2N-H \qquad (III)$$

worin $R^1$ und $R^2$ die oben genannte Bedeutung haben, umsetzt, oder

b) eine Verbindung der allgemeinen Formel IV

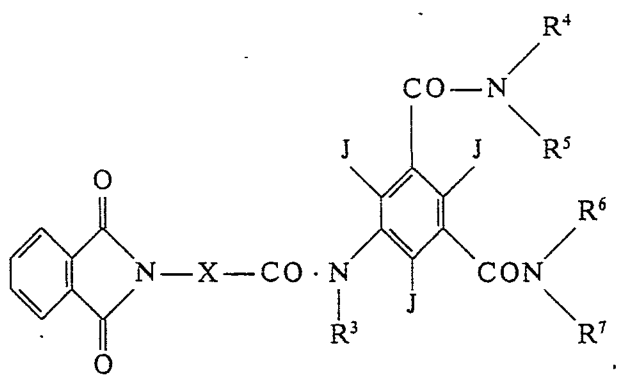

(IV)

worin
X und $R^3$–$R^7$ die oben angegebene Bedeutung haben, in an sich bekannter Weise der Hydrazinolyse unterwirft

und die so erhaltene Base gewünschtenfalls anschließend, gegebenenfalls unter intermediärem Schutz freier Hydroxylgruppen, N-alkyliert und/oder mit einer Röntgenkontrastmittelsäure in das Salz überführt.

Die Umsetzung des Ausgangsprodukts der allgemeinen Formel II mit dem Amin der Formel III (Verfahrensvariante a) erfolgt nach an sich bekannten Methoden in Gegenwart eines geeignten Lösungsmittels bei 0–100°C, vorzugsweise bei 0–60°C. Die Reaktionstemperatur wird im wesentlichen von den physikalischen Eigenschaften des eingesetzten Amins bestimmt. Ist das Amin leicht flüchtig, setzt man zweckmäßigerweise bei Raumtemperatur oder auch unterhalb

Raumtemperatur um. Ist das eingesetzte Amin weniger flüchtig, kann die Umsetzung auch bei höherer Reaktionstemperatur erfolgen. Geeignete Lösungsmittel sind insbesondere polare Lösungsmittel wie beispielsweise Wasser, Dioxan, Dimethylformamid, Dimethylacetamid, niedere Alkohole, wie zum Beispiel Methanol, Äthanol, Isopropanol u. a. und deren Gemische.

Verbindungen der Formel I, in denen $R^1$ und $R^2$ gleichzeitig Wasserstoff bedeuten, erhält man vorteilhaft gemäß Verfahrensvariante b) aus Verbindungen der allgemeinen Formel IV durch Hydrazinolyse. Die Durchführung der Hydrazinolyse erfolgt nach Methoden wie sie allgemein bekannt sind [vgl. z. B. DE-OS 2 523 567, J. Am. Chem. Soc. 71, 1856 (1949), J. Chem. Soc. 2343, 1926, Chem. Ber. 83, 244 (1950)]. Dazu wird das Ausgangsprodukt der allgemeinen Formel IV beispielsweise in einem organischen, vorzugsweise mit Wasser mischbaren Lösungsmittel (z. B. Methanol oder Äthanol) gelöst und mit Hydrazin in vorzugsweise alkoholischer Lösung zur Reaktion gebracht, wobei das Hydrazin zweckmäßigerweise im Überschuß angewandt wird. Die nachträgliche Einführung einer Alkyl- oder hydroxysubstituierten Alkylgruppe $R^3$ erfolgt ebenfalls nach den dem Fachmann an sich bekannten N-Alkylierungsmethoden, z. B. in dem man in alkalischer Lösung mit dem entsprechenden Alkylsulfat oder Alkylhalogenid, vorzugsweise Bromid, bei Raumtemperatur, umsetzt.

Ist es für den Reaktionsverlauf vorteilhaft vor der N-Alkylierung im Molekül anwesende freie Hydroxylgruppen intermediär zu schützen, so erfolgt dies nach üblichen Methoden durch leicht reversible Gruppen. Die Einführung solcher Schutzgruppen kann z. B. erfolgen durch Veresterung (z. B. Einführung eines vorzugsweisen Acetylrestes oder Benzylrestes) oder durch Verätherung (z. B. Einführung des Triphenylmethylrestes).

Der Hydroxylgruppenschutz kann auch durch Ketalisierung oder Acetalisierung, z. B. mittels Acetaldehyd, Aceton oder Dihydropyran, erreicht werden.

Die spätere Abspaltung der intermediär eingeführten Schutzgruppen unter Freisetzung der letztlich gewünschten Hydroxylgruppen erfolgt ebenfalls nach Methoden, die dem Fachmann allgemein geläufig sind. So kann die Abspaltung der Schutzgruppen ohne besondere Reaktionsstufe mit der Aufarbeitung und Isolierung der Umsetzungsprodukte erfolgen. Sie kann aber auch in üblicher Weise in einer getrennten Reaktionsstufe durchgeführt werden. Acylschutzgruppen können beispielsweise durch alkalische und Acetal-, Ketal- oder Ätherschutzgruppen durch saure Hydrolyse abgespalten werden.

Die Herstellung der erfindungsgemäß beschriebenen Salze erfolgt in an sich bekannter Weise, indem man z. B. die jodhaltige organische Säure mit der Base in Wasser in äquivalenten Gewichtsverhältnissen umsetzt. Aus der wäßrigen Lösung können die erfindungsgemäßen Salze nach den üblichen Methoden, wie beispielsweise durch Abdampfen des Lösungsmittels oder durch Fällung mit Hilfe organischer Lösungsmittel, isoliert werden.

Die neuen Ausgangsverbindungen der allgemeinen Formel II bzw. IV werden zweckmäßigerweise aus dem bekannten 5-Amino- bzw. 5-$R^3$-amino-2,4,6-trijod-isophthalsäuredichlorid (worin $R^3$ die oben angegebene Bedeutung hat) nach an sich bekannten Methoden hergestellt.

Im ersten Reaktionsschritt wird das 5-($R^3$-Amino)-2,4,6-trijod-isophthalsäuredihalogenid in üblicher Weise mit dem entsprechenden Halogenfettsäurechlorid Hal · X · COCl (worin Hal und X die oben angegebene Bedeutung haben) N-acyliert.

Im zweiten Reaktionsschritt erfolgt dann die Amidierung der beiden Carboxylgruppen durch Umsetzung des Säurechlorids mit HN · $R^4R^5$ bzw. HN · $R^6R^7$ nach an sich bekannten Methoden. So erfolgen beispielsweise die beiden Amidierungsreaktionen in einem geeigneten Lösungsmittel bei 0–100°C, vorzugsweise bei 20–80°C. Geeignete Lösungsmittel sind u. a. polare Lösungsmittel wie z. B. Wasser, Dioxan, Tetrahydrofuran, Dimethylformamid, Dimethylacetamid, Hexametapol u. ä. und deren Gemische. Da die Amidierungsreaktion exotherm verläuft, ist es gegebenenfalls zweckmäßig, das Reaktionsgemisch leicht zu kühlen, um die Reaktionstemperatur auf etwa 50°C bis 60°C halten zu können. Der während der Amidierungsreaktion freiwerdende Chlorwasserstoff wird entweder durch einen entsprechenden Überschuß an Base HN · $R^4R^5$ bzw. $HNR^6R^7$ oder durch Zusatz eines üblichen Protonenakzeptors gebunden. Als Protonenakzeptoren verwendet man vorteilhaft tertiäre Amine, wie z. B. Triäthylamin, Tributylamin oder Pyridin.

Die im Reaktionsverlauf anfallenden anorganischen oder organischen Salze werden in bekannter Weise abgetrennt, vorteilhaft z. B. mit Hilfe üblicher Ionenaustauscher-Säulen oder durch Filtration über bekannte Adsorbentien wie z. B. Diaion oder Amberlite XAD-2 und 4.

Im einzelnen sei die Herstellung des Ausgangsproduktes der Formel II am Beispiel für die Herstellung von 5-Chloracetamido-2,4,6-trijod-isophthalsäure-bis(N-methyl-2,3-dihydroxypropyl)-diamid erläutert:

Zu einer Lösung von 119,1 g (0,2 Mol) 5-Amino-2,4,6-trijod-isophthalsäuredichlorid in 400 ml Dimethylacetamid werden bei maximal 10°C innerhalb von 20 Minuten unter Rühren 67,7 g (0,6 Mol) Chloracetylchlorid zugetropft, 1 Stunde unter Eiskühlung und danach über Nacht bei Raumtemperatur weiterverrührt. Das Reaktionsgemisch wird dann mit 6 Liter Wasser behandelt, der entstandene Niederschlag abgesaugt und dieser in 3,5 Liter Essigester gelöst. Die Essigesterlösung wird mit 400 ml gesättigter $NaHCO_3$-Lösung und 2mal mit Wasser neutral gewaschen, mit $Na_2SO_4$ getrocknet und im Vakuum eingeengt. Der Rückstand wird mit 800 ml Äther behandelt und im Vakuum bei 50°C getrocknet. Man erhält so 100,8 g (75% der Theorie) 5-Chloracetamido-2,4,6-trijod-isophthalsäure-

dichlorid vom Schmelzpunkt 254 – 256° C (Zersetzung).

Zu einer Lösung von 490,7 g (0,73 Mol) 5-Chloracetamido-2,4,6-trijod-isophthalsäure-dichlorid in 5,8 Liter Dioxan werden unter Rühren bei Raumtemperatur innerhalb von 40 Minuten 322,3 g (3,07 Mol) N-Methylaminopropan-2,3-diol in 1,46 Liter Dioxan zugetropft und danach über Nacht weiterverrührt. Dann wird das Lösungsmittel abdestilliert, der Rückstand 2mal mit Dioxan gewaschen, in 1,1 Liter Wasser gelöst und über einen Kationenaustauscher und einem Anionenaustauscher filtriert. Nach Behandeln der wäßrigen Fraktion mit Aktivkohle wird im Vakuum eingeengt und der Rückstand bei 50° C im Vakuum getrocknet. Man erhält so 396 g (67%) 5-Chloracetamido-2,4,6-trijod-isophthalsäure-bis(N-methyl-2,3-dihydroxypropyl)-diamid vom Schmelzpunkt ~290° C (Zersetzung).

Die Herstellung des Ausgangsproduktes der allgemeinen Formel IV erfolgt gleichfalls aus 5-($R^3$-Amino)-2,4,6-trijod-isophthalsäure-dichlorid durch Acylierung der 5-ständigen aromatischen Aminogruppe mit dem entsprechenden N-Phthaloyl – X – COCl (X hat die oben angegebene Bedeutung) nach an sich bekannten Methoden und anschließender Amidierung der 1- und 3-ständigen Säurechloridgruppen wie oben beschrieben. Im einzelnen sei die Herstellung des Ausgangsproduktes der allgemeinen Formel IV am Beispiel der Herstellung von 2,4,6-Trijod-5-phthalimido-acetamido-isophthalsäure-bis(N-methyl-2,3-hydroxypropyl)-diamid dargestellt:

Zu einer eisgekühlten Lösung von 119,1 g (0,2 Mol) 5-Amino-2,4,6-trijod-isophthalsäure-dichlorid in 0,5 Liter Dimethylacetamid werden unter Rühren 178,9 g N-Phthaloylglycin-chlorid portionsweise zugefügt, einige Stunden unter Eiskühlung und danach über Nacht bei Raumtemperatur verrührt. Sodann wird die Reaktionslösung langsam in etwa 10 Liter Wasser eingerührt, 45 Minuten nachgerührt, der Niederschlag abgesaugt, dieser mit Wasser nachgewaschen und in 3 Liter Essigester aufgenommen. Die filtrierte Essigesterphase wird mit halbgesättigter Natriumbicarbonatlösung und 2mal mit Wasser gewaschen, mit Natriumsulfat getrocknet und im Vakuum eingeengt. Der Rückstand wird nachfolgend mit Äther behandelt und im Vakuum bei 50° C getrocknet. Man erhält so 127,3 g (81%) 2,4,6-Trijod-5-phthalimidoacetamido-isophthalsäure-dichlorid vom Schmelzpunkt ~255° C (Zersetzung).

Zu einer auf 50° C erwärmten Lösung von 78,3 g (0,1 Mol) 2,4,6-Trijod-5-phthalimidoacetamido-isophthalsäure-dichlorid in 0,25 Liter Dimethylacetamid werden unter Verrühren langsam eine Lösung von 31,5 g N-Methylaminopropan-2,3-diol in 0,15 Liter Dimethylacetamid zugetropft. Nach Abklingen der Wärmetönung werden sodann 71,3 ml Tributylamin zugefügt und eine weitere Stunde bei 50° C verrührt. Nach Abkühlen auf Raumtemperatur wird die Lösung im Vakuum eingeengt, der Rückstand 1 Stunde mit 2 Liter Methylenchlorid behandelt, der Niederschlag abgetrennt, mit Methylenchlorid nachgewaschen, nochmals mit 2 Liter Methylenchlorid nachbehandelt, die Substanz abgesaugt und im Vakuum bei 50° C getrocknet. Das Rohprodukt wird sodann unter Rühren mit Wasser erhitzt, wobei Kristallisation eintritt. Nach Erkalten unter Rühren wird der Niederschlag abgesaugt, mit Wasser nachgewaschen und bei 50° C im Vakuum getrocknet. Man erhält so 66,0 g (72%) 2,4,6-Trijod-5-phthalimido-acetamido-isophthalsäure-bis(N-methyl-2,3-hydroxypropyl)-diamid. Fp. 303° C (Zersetzung).

Definitionsgemäß können die Substituenten – CO – N · $R^4R^5$ und – $CONR^6R^7$ in den erfindungsgemäßen Verbindungen der Formel I auch verschieden sein. In diesem Falle erfolgt die Amidierung der 1- und 3-ständigen – COCl-Gruppen ebenfalls nach den oben angegebenen Arbeitsmethoden, jedoch stufenweise, d. h. zunächst mit der Base HN – $R^4R^5$ und anschließend mit der verschiedenen Base HN – $R^6R^7$, wie am Beispiel der Herstellung von 2,4,6-Trijod-5-phthalimidoacetamido-isophthalsäure-[(2,3-dihydroxypropyl)-(N-methyl-2,3-dihydroxypropyl)]-diamid im einzelnen ausgeführt sei:

Zu einer auf 60° C erwärmten Lösung von 42,1 g (0,4 Mol) N-Methylaminopropan-2,3-diol in 1 Liter Dioxan werden unter Rühren 156,6 g (0,2 Mol) 2,4,6-Trijod-5-phthalimidoacetamido-isophthalsäure-dichlorid zugefügt, 3 Stunden bei 60° C weiterverrührt, filtriert und das Filtrat im Vakuum eingeengt. Der Rückstand wird sodann 30 Minuten mit 2,5 Liter Wasser verrührt, der abgetrennte Niederschlag in 3 Liter Essigester mit je 1 Liter gesättigter $NaHCO_3$-Lösung und 1 Liter Wasser ausgeschüttelt, mit $Na_2SO_4$ getrocknet und im Vakuum eingeengt. Der Rückstand wird mit Äther nachbehandelt. Nach Trocknen im Vakuum bei 50° C erhält man so 108,4 g (64%) 2,4,6-Trijod-5-phthalimidoacetamido-isophthalsäure-(N-methyl-2,3-dihydroxypropyl)-amid-chlorid, Fp. 268 – 272° C (Zersetzung).

Zu einer auf 60° C erwärmten Lösung von 68,0 g 2,4,6-Trijod-5-phthalimidoacetamido-isophthalsäure-(N-methyl-2,3-dihydroxypropyl)-amid-chlorid in 1,5 Liter Dimethylacetamid werden unter Rühren tropfenweise 18,2 g 1-Amino-2,3-propandiol in 100 ml Dimethylacetamid zugefügt, 2 Stunden bei 50° C nachgerührt und das Dimethylacetamid im Vakuum entfernt. Der Rückstand wird zweimal mit Methylenchlorid behandelt, abgesaugt und im Vakuum bei 50° C getrocknet. Man erhält so 2,4,6-Trijod-5-phthalimidoacetamido-isophthalsäure-[(2,3-dihydroxypropyl)-(N-methyl-2,3-dihydroxypropyl)]-diamid, Ausbeute: 38,3 g (56%) Fp. 286 – 289° C (unter Zersetzung).

Beispiel 1

5-Aminoacetamido-2,4,6-trijod-
isophthalsäure-bis(N-methyl-2,3-dihydroxy-propyl)-diamid

50,0 g 5-Chloracetamido-2,4,6-trijod-isophthalsäure-bis(N-methyl-2,3-dihydroxypropyl)-diamid (Fp. 290°C unter Zersetzung) werden in 370 ml 25%igem Ammoniak unter Zusatz von 250 ml Wasser 7 Tage bei Raumtemperatur verrührt. Danach wird die Lösung eingeengt, mehrmals mit Wasser nachdestilliert, der Rückstand in 130 ml Wasser aufgenommen, die Lösung über einen Anionenaustauscher gegeben, das Filtrat mit Aktivkohle behandelt und im Vakuum eingeengt. Nach mehrmaligem Nachdestillieren mit Wasser wird das Präparat im Vakuum bei 50°C getrocknet. Ausbeute: 41,9 g (86%), Fp. ~259°C (unter Zersetzung).

In analoger Weise wurden hergestellt:

a) 5-(Aminoacetamido-N-methyl)-2,4,6-trijod-isophthalsäure-bis(N-methyl-2,3-dihydroxypropyl)-diamid. Ausbeute 95% der Theorie, Fp. 235°C (unter Zersetzung)

aus 5-(Chloracetamido-N-methyl)-2,4,6-trijod-isophthalsäure-bis(N-methyl-2,3-dihydroxy-propyl)-diamid, Fp. 305°C (Zersetzung)

b) 5-Aminoacetamido-2,4,6-trijod-isophthalsäure-bis(2,3-dihydroxypropyl)-diamid, Ausbeute 68% der Theorie, F. Fp. 244–246°C (Zersetzung)

aus 5-Chloracetamido-2,4,6-trijod-isophthalsäure-bis(2,3-dihydroxypropyl)-diamid, Fp. 313°C (Zersetzung)

c) 5-(Aminoacetamido-N-methyl)-2,4,6-trijod-isophthalsäure-bis(2,3-dihydroxypropyl)-diamid, Ausbeute 91% der Theorie, Fp. 235°C (Zersetzung)

aus 5-(Chloracetamido-N-methyl)-2,4,6-trijod-isophthalsäure-bis(2,3-dihydroxypropyl)-diamid, Fp. 235°C (Zersetzung)

d) 5-Aminoacetamido-2,4,6-trijod-isophthalsäure-bis(2-hydroxy-1-hydroxymethyl-äthyl)-diamid, Ausbeute 81% d. Th., Fp. 280°C (Zersetzung)

aus 5-Chloracetamido-2,4,6-trijod-isophthalsäure-bis(2-hydroxy-1-hydroxymethyl-äthyl)-diamid, Fp. 300°C (Zersetzung)

e) 5-(2-Aminopropionamido)-2,4,6-trijod-isophthalsäure-bis(2-hydroxy-1-hydroxymethyl-äthyl)-diamid, Ausbeute 39% d. Th., Fp. 278°C (Zersetzung)

aus 5-(2-Chlorpropionamido)-2,4,6-trijod-isophthalsäure-bis[2-hydroxy-1-hydroxymethyl-äthyl]-diamid, Fp. 314°C (Zersetzung)

f) 5-(3-Aminopropionamido)-2,4,6-trijod-isophthalsäure-bis(N-methyl-2,3-dihydroxypropyl)-diamid, Fp. 275°C (Zersetzung)

aus 5-(3-Chlorpropionamido)-2,4,6-trijod-isophthalsäure-bis(N-methyl-2,3-dihydroxypropyl)-diamid, Fp. 252°C (Zersetzung)

g) 5-(Aminoacetamido-N-methyl)-2,4,6-trijod-isophthalsäure-bis(2-hydroxy-1-hydroxymethyl-äthyl)-diamid, Ausbeute 57% d. Th., Fp. 263°C (Zersetzung)

aus 5-(Chloracetamido-N-methyl)-2,4,6-trijodisophthalsäure-bis(2-hydroxy-1-hydroxymethyl-äthyl)-diamid, Fp. 320°C (Zersetzung).

Beispiel 2

2,4,6-Trijod-5-methylaminoacetamido-
isophthalsäure-bis(N-methyl-2,3-dihydroxypropyl)-diamid

40,5 g (0,05 Mol) 5-Chloracetamido-2,4,6-trijod-isophthalsäure-bis(N-methyl-2,3-dihydroxypropyl)-diamid (Fp. 290°C, Zersetzung) werden mit 300 ml 40%iger wäßriger Methylaminolösung und Zusatz von 200 ml Wasser 7 Tage bei Raumtemperatur verrührt. Die Aufarbeitung des Reaktionsgemisches

erfolgt wie in Beispiel 1 beschrieben. Ausbeute 24,2 g (60%), Fp. ~245° C (unter Zersetzung).

In analoger Weise wurden hergestellt:

a) 2,4,6-Trijod-5-methylaminoacetamido-isophthalsäure-bis(2,3-dihydroxypropyl)-diamid, Ausbeute 73% d. Th., Fp. 265° C (Zersetzung)

aus 5-Chloracetamido-2,4,6-trijod-isophthalsäure-bis(2,3-dihydroxypropyl)-diamid, Fp. 313° C (Zers.)

b) 2,4,6-Trijod-5-(methylaminoacetamido-N-methyl)-isophthalsäure-bis(2,3-dihydroxypropyl)-diamid, Ausbeute 67% der Theorie, Fp. 265° C (Zersetzung)

aus 5-Chloracetamido-N-methyl-2,4,6-trijod-isophthalsäure-bis(2,3-dihydroxy-propyl)-diamid, Fp. 305° C (Zersetzung).

## Beispiel 3

### 5-[(2-Hydroxyäthyl)aminoacetamido]-2,4,6-trijod-isophthalsäure-bis(2-hydroxyäthyl)-diamid

In eine Lösung von 348,9 ml Äthanolamin in 375 ml Methanol werden unter Rühren 36,1 g 5-Chloracetamido-2,4,6-trijod-isophthalsäure-bis(2-hydroxyäthyl)-diamid (bis 320° C nicht geschmolzen bzw. zersetzt) eingetragen und 48 Stunden bei Raumtemperatur verrührt. Sodann wird im Vakuum eingeengt, der ölige Rückstand mit 187 ml Wasser über Nacht verrührt, der Niederschlag abgesaugt und im Vakuum bei 50° C getrocknet. Ausbeute 19,5 g (52%), Fp. 242 – 244° C (unter Zersetzung).

## Beispiel 4

### 5-[(2-Hydroxyäthyl)aminoacetamido]-2,4,6-trijod-isophthalsäure-bis(N-methyl-2,3-dihydroxypropyl)-diamid

Zu einer Lösung von 40,5 g 5-Chloracetamido-2,4,6-trijod-isophthalsäure-bis(N-methyl-2,3-dihydroxy-propyl)-diamid (Fp. 290°, Zersetzung) in 300 ml Methanol werden 299,5 ml Äthanolamin zugefügt, 48 Stunden bei Raumtemperatur verrührt. Die Lösung wird dann im Vakuum eingeengt, der Rückstand wird in 250 ml Wasser gelöst, die Lösung mit verdünnter Salzsäure auf pH 7 eingestellt, zur Entsalzung über Amberlite XAD-4 filtriert und die salzfreie Lösung nach Behandeln mit Aktivkohle im Vakuum eingeengt. Der Rückstand wird bei 50° C im Vakuum getrocknet. Ausbeute: 18,0 g (43%), Fp. ca. 250° C (unter Zersetzung).

## Beispiel 5

### 5-Aminoacetamido-2,4,6-trijod-isophthalsäure-bis(2-hydroxyäthyl)-diamid

50,4 g = 50 mMol 5-Bromacetamido-2,4,6-trijod-isophthalsäure-bis-(Bromacetoxy-äthylamid) werden in 500 ml 8 n Ammoniak suspendiert. Nach mehrstündigem Rühren wird die Lösung im Vakuum zur Trockne eingeengt, mit 150 ml Wasser versetzt, mit verdünntem Ammoniak auf pH 8 – 9 eingestellt und 1 – 2 Tage bis zur völligen Ausfällung gerührt. Ausbeute an Titelverbindung nach Absaugen, Waschen und Trocknen 25,5 g = 72,6% der Theorie. Zersetzung unter Jodabspaltung ab 270° C.

## Beispiel 6

### 5-Aminoacetamido-2,4,6-trijod-isophthalsäure-bis(N-methyl-2,3-dihydroxy-propyl)-diamid

Zu einer Suspension von 92,0 g 2,4,6-Trijod-5-phthalimido-acetamido-isophthalsäure-bis(N-methyl-2,3-dihydroxy-propyl)-diamid (Fp. ~303° C Zersetzung) in 600 ml Äthanol werden bei Raumtemperatur unter Rühren 18,2 ml 80%iges Hydrazinhydrat zugetropft und danach 2 Stunden am Rückfluß erwärmt. Nach dem Erkalten werden zur Reaktionsmischung 300 ml 1 n Salzsäure zugefügt. Anschließend wird das Reaktionsgemisch 1 Stunde bei Raumtemperatur gerührt, der Niederschlag abgesaugt, das Filtrat vom Äthanol im Vakuum befreit und die verbleibende wäßrige Lösung über einen basischen Ionenaustauscher filtriert. Das Filtrat wird dann im Vakuum eingeengt, der Rückstand mit 300 ml

n-Butanol unter Erwärmen am Rückfluß verrührt und bei Raumtemperatur nachgerührt. Der Niederschlag wird abgesaugt, mit kaltem n-Butanol nachbehandelt und im Vakuum bei 50°C getrocknet. Ausbeute: 62,4 g (79% der Theorie), Fp. ~263°C (unter Zersetzung).

In analoger Weise wurde hergestellt:

a) 5-Aminoacetamido-2,4,6-trijod-isophthalsäure-[(2,3-dihydroxypropyl)-(N-methyl-2,3-dihydroxypropyl)]-diamid, Fp. 251°C (Zersetzung)

aus 2,4,6-Trijod-5-phthalimidoacetamido-isophthalsäure-[(2,3-dihydroxypropyl)-(N-methyl-2,3-dihydroxypropyl)]-diamid, Fp. 286–289°C (Zersetzung)

b) 5-(2-Amino-propionamido)-2,4,6-trijod-isophthalsäure-bis(2,3-dihydroxypropyl)-diamid; Ausbeute 49% der Theorie, Fp.: 248°C (Zersetzung)

aus 2,4,6-Trijod-5-phthalimidopropionamido-isophthalsäure-bis(2,3-dihydroxypropyl)-diamid, Fp.: ~298°C (Zersetzung)

c) 5-(3-Amino-propionamido)-2,4,6-trijod-isophthalsäure-bis(2,3-dihydroxypropyl)-diamid; Ausbeute 26% der Theorie; Fp.: 278–281°C (Zersetzung)

aus 2,4,6-Trijod-5-phthalimidopropionamido-isophthalsäure-bis(2,3-dihydroxypropyl)-diamid, Fp.: 298–300°C (Zersetzung)

d) 5-Aminoacetamido-2,4,6-trijod-isophthalsäure-tetrakis-(2-hydroxyäthyl)-diamid; Ausbeute 44% der Theorie; Fp. 262–263°C (Zersetzung)

aus 2,4,6-Trijod-5-phthalimido-acetamido-isophthalsäure-tetrakis(2-hydroxyäthyl)-diamid; Fp.: 283–285°C (Zersetzung)

## Beispiel 7

### 5-Aminoacetamido-2,4,6-trijod-isophthalsäure-bis(N-methyl-2,3-dihydroxy-propyl)-diamid-Salz der Iothalamsäure

| | |
|---|---|
| 5-Aminoacetamido-2,4,6-trijod-isophthalsäure-bis(N-methyl-2,3-dihydroxypropyl)-diamid | 394,3 g |
| 5-Acetamido-2,4,6-trijod-isophthalsäure-monomethylamid (Iothalamsäure) | 306,4 g |
| Calzium-dinatriumadetat | 0,1 g |
| bidestilliertes Wasser | ad 1000 ml |

Vor dem Auffüllen wird die Lösung gegebenenfalls durch Zugabe einer geringen Menge der entsprechenden Säure bzw. Base auf einen pH von 6,5–7,5 eingestellt, die Lösung durch ein Bakterienfilter filtriert, abgefüllt und sterilisiert. Sie enthält 380 mg J/ml.

## Beispiel 8

### 5-Aminoacetamido-2,4,6-trijod-isophthalsäure-bis(N-methyl-2,3-dihydroxypropyl)-diamid-Salz der 5,5'-(3,6-Dioxaoctandioyl-diimino)-bis[2,4,6-trijod-3-(2-methoxy-1-methylcarbamoyl-äthyl)-isophthalamsäure

| | |
|---|---|
| 5-Aminoacetamido-2,4,6-trijod-isophthalsäure-bis(N-methyl-2,3-dihydroxypropyl)-diamid | 39,4 g |
| 5,5'-(3,6-Dioxaoctandioyl-diimino)-bis[2,4,6-trijod-3-(2-methoxy-1-methylcarbamoyl-äthyl)-isophthalamsäure | 37,1 g |
| Calziumdinatriumedetat | 0,01 g |
| bidestilliertes Wasser | ad 100 ml |

Vor dem Auffüllen wird die Lösung gegebenenfalls durch Zugabe einer geringen Menge der entsprechenden Säure bzw. Base auf einen pH von 6,5–7,5 eingestellt, die Lösung durch ein Bakterienfilter filtriert, abgefüllt und sterilisiert. Sie enthält 380 ml J/ml.

Beispiel 9

2,4,6-Trijod-5-methylaminoacetamido-isophthalsäure-bis(N-methyl-2,3-dihydroxypropyl)-diamid-Salz der Iothalamsäure

| | |
|---|---|
| 2,4,6-Trijod-5-methylaminoacetamido-isophthalsäure-bis(N-methyl-2,3-dihydroxypropyl)-diamid | 31,7 g |
| 5-Acetamido-2,4,6-trijod-isophthalsäure-monomethylamid (Iothalamsäure) | 24,2 g |
| Calzium-dinatriumedetat | 0,01 g |
| bidestilliertes Wasser | ad 100 ml |

Vor dem Auffüllen wird die Lösung gegebenenfalls durch Zugabe einer geringen Menge der entsprechenden Säure bzw. Base auf einen pH von 6,5—7,5 eingestellt, die Lösung durch ein Bakterienfilter filtriert, abgefüllt und sterilisiert. Sie enthält 300 mg J/ml.

Beispiel 10

5-Aminoacetamido-2,4,6-trijod-isophthalsäure-bis(2-hydroxyäthyl)-diamid-Salze der Iothalamsäure

| | |
|---|---|
| 5-Aminoacetamido-2,4,6-trijod-isophthalsäure-bis(2-hydroxyäthyl)-diamid | 27,7 g |
| 5-Acetamido-2,4,6-trijod-isophthalsäure-monomethylamid (Iothalamsäure) | 24,2 g |
| Calzium-dinatriumedetat | 10 mg |
| bidestilliertes Wasser | ad 100 ml |

Vor dem Auffüllen wird die Lösung gegebenenfalls durch Zugabe einer geringen Menge der entsprechenden Säure bzw. Base auf einen pH von 6,5—7,5 eingestellt. Anschließend wird die Lösung über einem Bakterienfilter filtriert, abgefüllt und sterilisiert. Sie enthält 300 mg J/ml Lösung.

Beispiel 11

5-Aminoacetamido-2,4,6-trijod-isophthalsäure-bis(N-methyl-2,3-dihydroxypropyl)-diamid-Salz der Amidotrizoe-Säure

Wie im Beispiel 8 beschrieben, erhält man aus 5-Aminoacetamido-2,4,6-trijod-isophthalsäure-bis(N-methyl-2,3-dihydroxypropyl)-diamid und Amidotrizoesäure die entsprechende Kontrastmittellösung mit einem Jodgehalt von 380 mg J/ml.

**Patentansprüche**

1. 5-Amino-2,4,6-trijod-isophthalsäure-bis-amide der allgemeinen Formel I

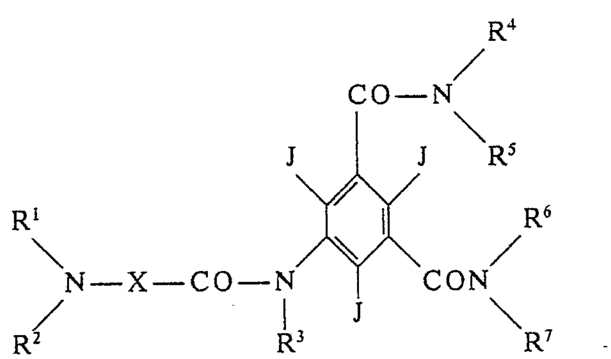

(I)

worin bedeuten

X ein gerad- oder verzweigtkettiger, gegebenenfalls durch eine Hydroxy-, Methoxy- oder Aminogruppe substituierter Alkylenrest mit 1 bis 4 Kohlenstoffatomen,

$R_1$ und $R_3$ Wasserstoff oder einen Alkylrest mit 1 bis 4 Kohlenstoffatomen,
$R_2$ Wasserstoff oder einen hydroxylierten Alkylrest mit 1 bis 4 Kohlenstoffatomen,
$R_4$ und $R_6$ Wasserstoff oder einen gegebenenfalls hydroxylierten Alkylrest mit 1 bis 4 Kohlenstoffatomen,
$R_5$ und $R_7$ einen Mono- oder Polyhydroxyalkylrest mit 1 bis 4 Kohlenstoffatomen,
und deren Salze mit Kontrastmittelsäuren.

2. 5-Aminoacetamido-2,4,6-trijod-isophthalsäure-bis-(2-hydroxyäthyl)-diamid.
3. 5-Aminoacetamido-2,4,6-trijod-isophthalsäure-bis-(N-methyl-2,3-dihydroxypropyl)-diamid.
4. 5-Aminoacetamido-2,4,6-trijod-isophthalsäure-bis-(2-hydroxyäthyl)-diamid-Salze der Iothalamsäure.
5. 5-Aminoacetamido-2,4,6-trijod-isophthalsäure-bis-(N-methyl-2,3-dihydroxypropyl)-diamid-Salze der Iothalamsäure.
6. 5-Aminoacetamido-2,4,6-trijod-isophthalsäure-bis(N-methyl-2,3-dihydroxypropyl)-diamid-Salz der Amidotrizoesäure.
7. Verfahren zur Herstellung der Verbindungen der allgemeinen Formel (I), dadurch gekennzeichnet, daß man in an sich bekannter Weise

a) eine Verbindung der allgemeinen Formel II

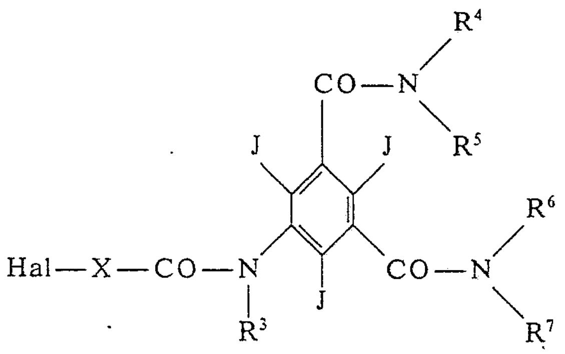

(II)

worin $R^3$ bis $R^7$ und X die oben angegebene Bedeutung haben und Hal ein Chlor- oder Bromatom darstellt, mit einem Amin der allgemeinen Formel III

$$R^1R^2N-H \qquad (III)$$

worin $R^1$ und $R^2$ die oben genannte Bedeutung haben, umsetzt, oder

b) eine Verbindung der allgemeinen Formel IV

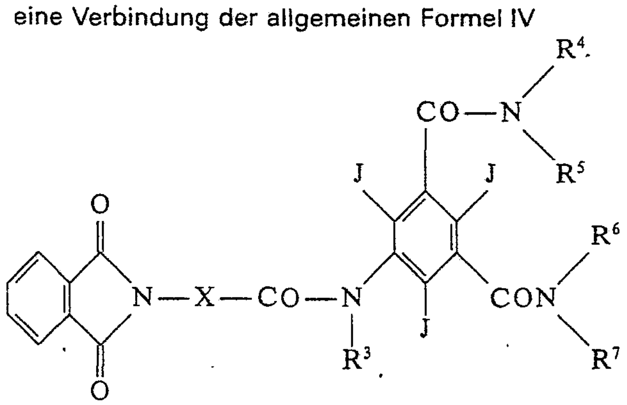

(IV)

worin X und $R^3$–$R^7$ die oben angegebene Bedeutung haben, in an sich bekannter Weise der Hydrazinolyse unterwirft

und die so erhaltene Base gewünschtenfalls anschließend, gegebenenfalls unter intermediärem Schutz freier Hydroxygruppen N-alkyliert und/oder mit einer Röntgenkontrastmittelsäure in das Salz überführt.

8. Röntgenkontrastmittel, enthaltend als schattengebende Substanz Salze von Basen gemäß Anspruch 1 und Röntgenkontrastmittelsäuren.

9. Röntgenkontrastmittel, enthaltend als schattengebende Substanz mindestens eine Verbindung gemäß Anspruch 1–6.

## Claims

1. 5-amino-2,4,6-triiodoisophthalic acid bis-amides of the general formula I

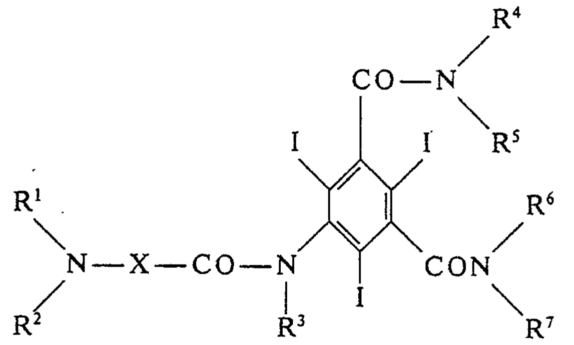

(I)

in which

X represents a straight-chain or branched alkenyl radical having from 1 to 4 carbon atoms which is optionally substituted by a hydroxy, methoxy or amino group,

$R_1$ and $R_3$ represent hydrogen or an alkyl radical having from 1 to 4 carbon atoms,

$R_2$ represents hydrogen or a hydroxylated alkyl radical having from 1 to 4 carbon atoms,

$R_4$ and $R_6$ represent hydrogen or an optionally hydroxylated alkyl radical having from 1 to 4 carbon atoms, and

$R_5$ and $R_7$ represent a mono- or poly-hydroxyalkyl radical having from 1 to 4 carbon atoms, and the salts thereof with contrast agent acids.

2. 5-aminoacetamido-2,4,6-triiodoisophthalic acid bis-(2-hydroxyethyl)-diamide.
3. 5-aminoacetamido-2,4,6-triiodoisophthalic acid bis(N-methyl-2,3-dihydroxypropyl)-diamide.
4. 5-aminoacetamido-2,4,6-triiodoisophthalic acid bis(2-hydroxyethyl)-diamide salts of iothalamic acid.
5. 5-aminoacetamido-2,4,6-triiodoisophthalic acid bis(N-methyl-2,3-dihydroxypropyl)-diamide salts of iothalamic acid.
6. 5-aminoacetamido-2,4,6-triiodoisophthalic acid bis(N-methyl-2,3-dihydroxypropyl)-diamide salt of amidotrizoic acid.
7. Process for the manufacture of compounds of the general formula (I), characterised in that, in a manner known per se,

a) a compound of the general formula II

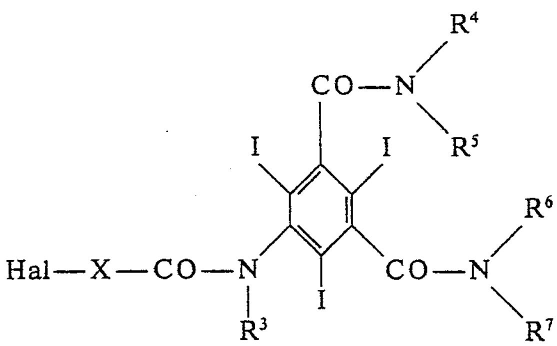

(II)

in which $R^3$ to $R^7$ and X have the meanings given above and Hal represents a chlorine or bromine atom, is reacted with an amine of the general formula III

$$R^1R^2N-H \qquad (III)$$

in which $R^1$ and $R^2$ have the meanings given above, or

b) a compound of the general formula (IV)

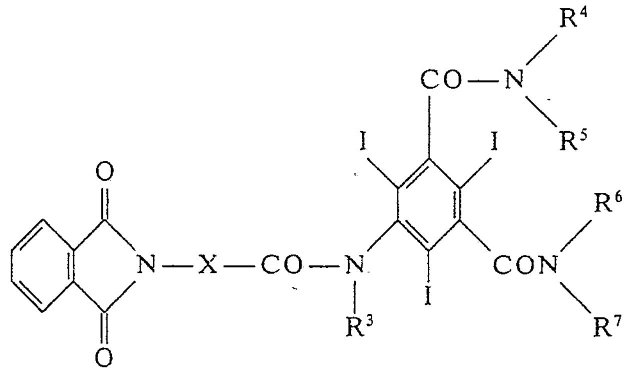

(IV)

in which X and $R^3$—$R^7$ have the meanings given above,

is subjected, in a manner known per se, to hydrazinolysis, and the base obtained in this manner is, if desired, N-alkylated, optionally with the temporary protection of free hydroxy groups, and/or is converted into the salt with an X-ray contrast agent acid.

8. X-ray contrast agents containing, as radio-opaque substance, salts of bases according to claim 1 and X-ray contrast agent acids.

9. X-ray contrast agents containing, as radio-opaque substance, at least one compound according to claims 1 to 6.

**Revendications**

1. Bis-amides d'acides amino-5 triiodo-2,4,6 isophtaliques répondant à la formule générale I

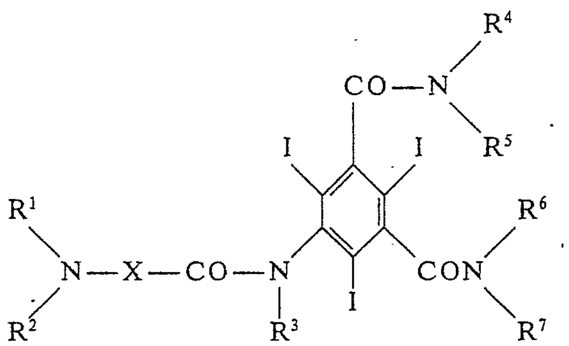

(I)

dans laquelle

X représente un radical alkylène contenant de 1 à 4 atomes de carbone, linéaire ou ramifié, éventuellement porteur d'un radical hydroxy, méthoxy ou amino,

$R_1$ et $R_3$ représentent chacun l'hydrogène ou un radical alkyle contenant de 1 à 4 atomes de carbone,

$R_2$ représente l'hydrogène ou un radical alkyle hydroxylé contenant de 1 à 4 atomes de carbone,

$R_4$ et $R_6$ représentent chacun l'hydrogène ou un radical alkyle contenant de 1 à 4 atomes de carbone et éventuellement hydroxylé, et

$R_5$ et $R_7$ représentent chacun un radical monohydroxy- ou polyhydroxy-alkyle contenant de 1 à 4 atomes de carbone,

ainsi que les sels qu'ils forment avec des agents de contraste qui sont des acides.

2. Bis-[(hydroxy-2 éthyl)-amide] de l'acide amino-acétylamino-5 triiodo-2,4,6 isophtalique.

3. Bis-[N-méthyl-N-(dihydroxy-2,3 propyl)-amide] de l'acide amino-acétylamino-5 triiodo-2,4,6-isophtalique.

4. Sels formés par l'acide iotalamique et le bis-[(hydroxy-2 éthyl)-amide] de l'acide amino-acétylamino-5 triiodo-2,4,6 isophtalique.

5. Sels formés par l'acide iotalamique et le bis-[N-méthyl-N-(dihydroxy-2,3 propyl)-amide] de l'acide amino-acétylamino-5 triiodo-2,4,6 isophtalique.

6. Sel formé par l'acide amido-trizoïque et le bis-[N-méthyl-N-(dihydroxy-2,3 propyl)-amide] de l'acide amino-acétylamino-5 triiodo-2,4,6 isophtalique.

7. Procédé de préparation des composés de formule générale I, procédé caractérisé en ce que, en opérant de manière connue:

a) on fait réagir un composé répondant à la formule générale II

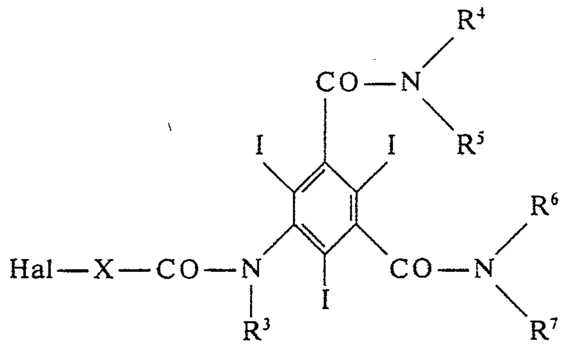

(II)

dans laquelle les symboles $R^3$ à $R^7$ et X ont les significations indiquées plus haut et Hal représente un atome de chlore ou de brome, avec une amine répondant à la formule générale III

$$R^1R^2N—H \qquad (III)$$

dans laquelle $R^1$ et $R^2$ ont les significations précédemment données, ou

b) on soument à une hydrazinolyse, de manière connue, un composé répondant à la formule générale IV

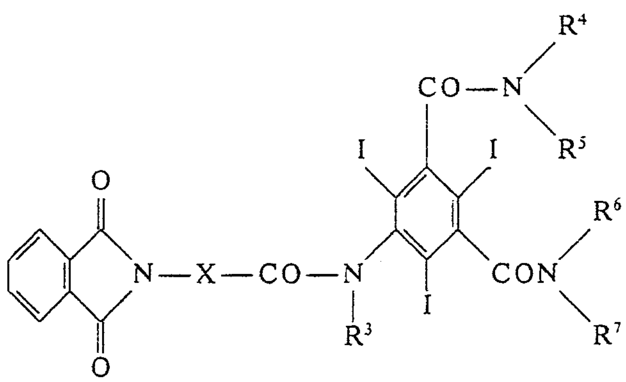

(IV)

dans laquelle X et $R^3$ à $R^7$ ont les significations précédemment données,

après quoi, si on le désire, on alkyle à l'azote la base ainsi obtenue, éventuellement en protégeant intermédiairement les groupes hydroxy libres, et/ou on la transforme en sel avec un acide approprié qui est un agent de contraste radiologique.

8. Agent de contraste radiologique contenant, comme substance opacifiante, un sel formé par une base selon la revendication 1 et par un acide qui est un agent de contraste.

9. Agent de contraste contenant, comme substance opacifiante, au moins un composé selon l'une quelconque des revendications 1 à 6.